Europäisches Patentamt

European Patent Office

Office européen des brevets

(19)

(11) **EP 0 776 181 B1**

## (12) FASCICULE DE BREVET EUROPEEN

(45) Date de publication et mention
de la délivrance du brevet:
**28.02.2001 Bulletin 2001/09**

(21) Numéro de dépôt: **94926281.0**

(22) Date de dépôt: **06.09.1994**

(51) Int Cl.⁷: **A61B 19/00**, G02B 21/24,
G01B 9/04

(86) Numéro de dépôt international:
**PCT/FR94/01049**

(87) Numéro de publication internationale:
**WO 95/07054 (16.03.1995 Gazette 1995/12)**

(54) **DISPOSITIF DE DESIGNATION OPTIQUE, NOTAMMENT POUR OPERATION DE MICROCHIRURGIE**

OPTISCHE ANZEIGEVORRICHTUNG, INSBESONDERE FUER MIKROCHIRURGISCHE OPERATIONEN

OPTICAL DESIGNATING DEVICE, ESPECIALLY FOR USE IN MICROSURGERY

(84) Etats contractants désignés:
**AT BE CH DE DK ES FR GB GR IE IT LI LU MC NL PT SE**

(30) Priorité: **07.09.1993 FR 9310625**

(43) Date de publication de la demande:
**04.06.1997 Bulletin 1997/23**

(73) Titulaire: **Medtronic Inc.**
**Minneapolis, MN 55432 (US)**

(72) Inventeur: **DRUAIS, Hervé**
**F-38000 Grenoble (FR)**

(74) Mandataire: **Pernez, Helga**
**Breese-Majerowicz,**
**3, avenue de l'Opéra**
**75001 Paris (FR)**

(56) Documents cités:
WO-A-88/08282     FR-A- 2 682 778
US-A- 3 710 798     US-A- 5 143 076

EP 0 776 181 B1

## Description

La présente invention concerne un dispositif de désignation optique, notamment pour la réalisation d'opérations de microchirurgie. Un tel dispositif est destiné à être associé à un instrument optique tel qu'un microscope ou un binoculaire. Dans la suite, on désignera par "microscope" tout instrument optique employé pour l'observation de la zone d'intervention en chirurgie et en microchirurgie.

De tels microscopes sont employés pour permettre au chirurgien d'effectuer des manipulations de précision. Un des problèmes est de repérer une cible dans le champ de vision du microscope. Un autre problème est d'exploiter les informations obtenues par des techniques d'imagerie par exemple la tomodensitométrie et la résonance magnétique nucléaire, pour guider les gestes du chirurgien.

On connaît dans l'art antérieur un système de localisation décrit dans le brevet US5143076. Ce système comporte un cadre solidaire de la tête du patient. Ce cadre supporte des sources lumineuses dont l'intersection désigne une zone particulière de la tête du patient, par exemple la cible. Ce système n'est pas totalement satisfaisant car il ne permet pas de tenir compte de manière simple des déplacements du microscope, et limite les voies d'accès du fait de la présence du cadre supportant les sources lumineuses.

On connaît par exemple dans l'état de la technique le brevet français FR2682778 concernant un microscope pour opération de microchirurgie stéréotaxique assistée par ordinateur. Ce document décrit un microscope pour opération de microchirurgie stéréotaxique assistée par ordinateur, et un procédé pour son fonctionnement. Ce microscope comporte des détecteurs détectant des données optiques, un système d'identification de position et un dispositif de commande de processus évaluant les signaux dudit système, ce système est un système à base optique intégré dans le système optique du microscope. Il est prévu un dispositif qui convertit les signaux délivrés par le dispositif en une représentation graphique bidimensionnelle. Un tel microscope présente une grande complexité de mise en oeuvre, et n'est pas adapté au travail quotidien du chirurgien.

On connaît également le brevet WO88/08282 Appareil permettant de délimiter la position d'une cible constituant une partie située dans le cerveau d'un patient, devant être traité chirurgicalement, et faisant appel à la tomographie assistée par ordinateur. Le dispositif de l'art antérieur comprend un cadre pouvant être fixé de manière inamovible selon une façon connue en soi, à la boîte crânienne du patient.

Un premier but de la présente invention est de proposer un moyen simple permettant au chirurgien de surveiller l'approche d'une zone critique, ou cible, sans quitter l'oculaire du microscope.

Un second but de l'invention est de permettre le calcul de la distance entre la surface observée et la cible de façon à déterminer la profondeur entre la cible par rapport à la surface du champs opératoire.

Un troisième but de l'invention est de permettre un asservissement des moyens de repérage pour permettre un suivi de la cible nonobstant les mouvements relatifs du microscope par rapport au champ observé, et éventuellement un asservissement des moyens de désignation par rapport à une base d'images préenregistrées.

L'invention concerne un dispositif de désignation optique conforme à la revendication principale.

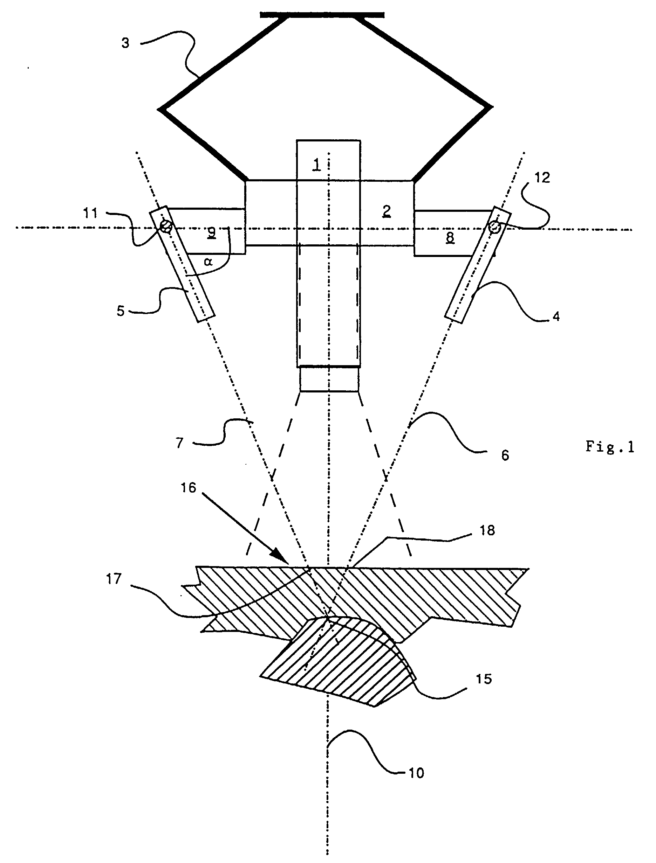

L'invention sera mieux comprise à la lecture de la description qui suit, faisant référence aux dessins annexés où la figure unique représente une vue schématique du dispositif de désignation optique.

La figure unique représente une vue schématique du dispositif de désignation optique selon l'invention. Il comporte un instrument optique (1) de type connu, monté sur une platine porte-outil (2) solidaire d'un support articulé (3).

Le dispositif comporte dans l'exemple décrit deux sources de lumière (4, 5), par exemple de type LASER, solidaires de la platine porte-outil (2). Les deux sources lumineuses (4, 5) émettent des faisceaux faiblement divergent dont les axes optiques sont référencés (6, 7).

Les sources optiques sont disposées sur des extensions (8, 9) de la platine porte-outil (2) supportant l'instrument optique (1) de façon à décaler les deux sources lumineuses symétriquement par rapport à l'axe optique principal (10).

Les sources lumineuses (4, 5) sont mobiles angulairement par rapport à des pivots (11, 12) tangentiels par rapport à un disque fictif ayant pour axe de symétrie l'axe optique principal (10). Le positionnement des sources lumineuses peut être assuré manuellement, mais de préférence il est motorisé. A cet effet, le dispositif comporte pour chacune des sources lumineuses (4, 5) un moteur électrique et le cas échéant un ensemble réducteur assurant le déplacement angulaire des sources lumineuses (4, 5) en fonction de signaux d'asservissement provenant d'un calculateur recevant des informations provenant d'une base de données Image dans laquelle sont enregistrées les images provenant d'un système d'imagerie, et permettant de calculer la position d'une cible dans le référentiel du patient, et ou recevant des informations provenant de capteurs de recopie de la position du support articulé (2), et permettant de calculer la position du microscope dans un référentiel fixe.

A défaut, l'orientation angulaire des sources lumineuses (4, 5) est déterminée de façon à converger en un point-cible (15).

Le point-cible (15) peut par exemple être constitué par la surface d'un vaisseau sanguin, d'un nerf ou d'un organe qu'il convient de ne pas atteindre lors de l'intervention chirurgicale.

Le décalage entre la surface (16) observée par l'opérateur, et le point-cible (15) se traduit par un décalage des deux spots (17, 18) visibles à l'intersection entre la surface observée (16) et les faisceaux lumineux (6, 7). Ce décalage se réduit au fur et à mesure que l'on se rapproche du point-cible (15).

Par ailleurs, la distance entre les deux spots (17, 18) permet de déduire la profondeur du point-cible (15) par rapport à la surface observée (16), par un calcul de triangulation:

$$p = (^E/_2 * tg\ \alpha) - L$$

Où

- P désigne la profondeur de la cible par rapport à la surface observée
- E désigne l'écartement des deux sources lumineuses
- $\alpha$ désigne l'angle formé par les faisceaux lumineux (6, 7) par rapport à un plan perpendiculaire à l'axe optique principal (10)
- L désigne distance entre le plan des deux sources lumineuses (4, 5), et la surface observée.

Cette distance L est déterminée par les caractéristiques et réglages du microscope, puisqu'il est fonction de la distance entre l'objectif et la surface observée, et donc du réglage du système optique permettant d'obtenir une image nette.

Le résultat de ce calcul peut être visualisé dans le viseur du système optique, ou sur tout système d'affichage de type connu. Selon un troisième mode d'utilisation, on réalise une mesure de la profondeur de la cible (15), sans connaissance préalable de la distance entre la surface observée et l'instrument optique.

A cet effet, la distance entre la surface observée et le plan passant par les deux sources lumineuses (4, 5) est déterminé en faisant converger les faisceaux (6, 7) en un point unique de la surface observée (18), de façon à superposer les deux spots (17, 18).

On enregistre alors l'orientation angulaire $\alpha_1$ des faisceaux optiques. L'étape suivante consiste à commander l'orientation des faisceaux (6, 7) de façon à les faire en un point (15) de la cible. Les faisceaux présentent alors une nouvelle orientation $\alpha$ qui permet de déterminer la profondeur de la cible par rapport à la surface observée en application de l'équation suivante:

$$P = \frac{E\ (tg\ \alpha - tg\ \alpha_1)}{2}$$

Où

- P désigne la profondeur de la cible par rapport à la surface observée

- E désigne l'écartement des deux sources lumineuses
- $\alpha$ désigne l'angle formé par les faisceaux lumineux (6, 7) par rapport à un plan perpendiculaire à l'axe optique principal (10).
- $\alpha_1$ désigne l'angle initial formé par les faisceaux lumineux (6, 7) par rapport à un plan perpendiculaire à l'axe optique principal (10), et assurant la convergence des deux spots (17, 18) sur la surface observée.

Il est également possible de procéder à une évaluation de la profondeur de la cible à partir de l'écartement e des deux spots lumineux (17, 18). La profondeur de la cible est dans ce cas déterminée comme suit:

$$P = e\ \frac{tg\ \alpha}{2}$$

Où

- P désigne la profondeur de la cible par rapport à la surface observée
- e désigne l'écartement des deux spots lumineux
- $\alpha$ désigne l'angle formé par les faisceaux lumineux (6, 7) par rapport à un plan perpendiculaire à l'axe optique principal (10).

L'écartement e des spots lumineux peut être mesuré ou évalué de façon connue par une grille calibrée visible à travers l'instrument optique, ou par tout autre moyen équivalent.

Le dispositif selon l'invention permet de surveillance visuellement l'approche du point-cible, et en outre d'afficher la distance calculée. Le chirurgien peut ainsi adapter de façon optimale ses gestes opératoires.

Selon un autre mode d'utilisation, on réalise un suivi de la cible. Lorsque la profondeur de la cible est connue par l'une des méthodes précédentes, il est possible de commander l'orientation angulaire des sources lumineuses (4, 5) en appliquant la formule trigonométrique suivante:

$$\alpha = Arctg\ (\frac{2L+p}{E})$$

Après la détermination de l'orientation angulaire initiale, il est possible d'asservir la variation angulaire aux déplacements du système optique dont l'utilisateur cherchera à conserver la focalisation sur la surface observée, ce qui permet de déduire l'évolution de la profondeur de la cible au fur et à mesure de la progression de l'intervention chirurgicale.

La détermination de la position angulaire des sources lumineuses (4, 5) est assurée par des capteurs angulaires électriques, électromécaniques ou optroniques de type connu. Ils délivrent des signaux électriques mis en oeuvre par un calculateur pour le recalcul de l'orientation de chacun des faisceaux lumineux en fonction des déplacements du support (2) dans le référentiel fixe.

A titre d'exemple, le dispositif est utilisé selon des modes successifs:

- acquisition de l'orientation initiale des sources lumineuses dirigées de façon à assurer la convergences des spots sur la surface observée;
- déplacement initial des sources lumineuses pour désigner la cible (15) ;
- déplacements des sources lumineuses asservis aux mouvements de l'instrument optique.

L'invention est décrite dans ce qui précède à titre d'exemple non limitatif. Il est bien entendu que l'Homme de Métier sera à même de proposer de nombreuses variations et adaptation sans pour autant sortir du cadre de l'invention. En particulier, l'exemple de réalisation décrit comporte deux spots disposés symétriquement dans le plan perpendiculaire à l'axe d'observation du microscope. Il est bien entendu possible de prévoir un nombre différent de spots, et de disposer ces spots de façon asymétrique.

## Revendications

1. Dispositif de désignation optique pour opération de microchirurgie comportant une platine porte-outil (2) solidaire d'un instrument optique (1), supportant deux sources lumineuses (4, 5) décalées par rapport à l'axe optique (10) de l'instrument optique (1), lesdites sources optiques étant orientées de façon à diriger des faisceaux lumineux (6, 7) en direction d'un point-cible, caractérisé en ce que le point-cible est dans le référentiel du patient, en ce que l'instrument optique (1) et la platine porte-outil (2) sont solidaires d'un support articulé (3) mobile par rapport à un référentiel fixe, en ce que l'orientation des deux sources (4, 5) est déterminée par rapport à l'axe optique de l'instrument optique (1), en ce qu'il comporte un calculateur et en ce qu'il comporte pour chacune des sources lumineuses (4, 5) un moteur électrique assurant le déplacement angulaire des sources lumineuses (4, 5) commandé par le calculateur afin de faire converger les faisceaux (6, 7) vers le point-cible (15), le calculateur assurant le recalcul de l'orientation de chacun des faisceaux (6, 7) en fonction des déplacements du support dans le référentiel fixe.

2. Dispositif de désignation optique pour opération de microchirurgie selon la revendication 1 caractérisé en ce que le calculateur commande les moteurs assurant le déplacement angulaire des sources lumineuses (4, 5) de façon à assurer la convergence des faisceaux lumineux (6, 7) vers le point-cible (15) dont les coordonnées sont connues dans le référentiel du champ observé.

3. Dispositif de désignation optique pour opération de microchirurgie selon la revendication 1 caractérisé en ce qu'il comporte en outre des capteurs de position angulaire délivrant un signal fonction de l'orientation de chacune des sources lumineuses et un calculateur pour la détermination de la profondeur du point-cible (15).

4. Dispositif de désignation optique pour opération de microchirurgie selon l'une quelconque des revendications précédentes caractérisé en ce qu'il comporte en outre un calculateur commandant les moteurs de positionnement des sources lumineuses (4, 5) de façon à compenser les déplacements du système optique (1) par rapport au référentiel fixe.

## Patentansprüche

1. Optische Bezeichnungsvorrichtung für Mikrochirurgie-Operationen mit einer Werkzeugträgerplatte (2), die mit einem optischen Instrument (1) verbunden ist und zwei in Bezug auf die Optikachse (10) des optischen Instruments (1) versetzt angeordnete Lichtquellen (4, 5) trägt, wobei die besagten Optikquellen so ausgerichtet sind, dass sie Lichtstrahlen (6, 7) in Richtung eines Zielpunktes abgeben, dadurch gekennzeichnet, dass sich der Zielpunkt unter den Bezugswerten des Patienten befindet, dass das optische Instrument (1) und die Werkzeugträgerplatte (2) mit einem gelenkigen, in Bezug auf einen festen Bezugswert mobilen Träger (3) verbunden sind, dass die Ausrichtung der beiden Quellen (4, 5) in Bezug auf die Optikachse des optischen Instruments (1) definiert ist, dass sie einen Rechner umfasst, und dass sie für jede der Lichtquellen (4, 5) einen Elektromotor aufweist, der die vom Rechner gesteuerte Winkelverschiebung der Lichtquellen (4, 5) gewährleistet, um die Strahlen (6, 7) zu einem Zielpunkt (15) hin in Übereinstimmung zu bringen, wobei der Rechner die Neuberechnung der Ausrichtung eines jeden Strahls (6, 7) entsprechend den Verschiebungen des Trägers nach den festen Bezugswerten gewährleistet.

2. Optische Bezeichnungsvorrichtung für Mikrochirurgie-Operationen nach Anspruch 1, dadurch gekennzeichnet, dass der Rechner die die Winkelverschiebung der Lichtquellen (4, 5) gewährleistenden Motoren so steuert, dass die Konvergenz der Lichtstrahlen (6, 7) zum Zielpunkt (15) gewährleistet wird, dessen Koordinaten unter den Bezugswerten des betrachteten Feldes bekannt sind.

3. Optische Bezeichnungsvorrichtung für Mikrochirurgie-Operationen nach Anspruch 1, dadurch gekennzeichnet, dass sie des weiteren Winkelpositionsgeber aufweist, die ein Signal entsprechend der Ausrichtung jeder Lichtquelle abgeben, sowie einen Rechner für die Ermittlung der Tiefe des Zielpunktes (15).

4. Optische Bezeichnungsvorrichtung für Mikrochirurgie-Operationen nach einem der vorstehend genannten Ansprüche, dadurch gekennzeichnet, dass sie des weiteren einen Rechner umfasst, der die Positioniermotoren der Lichtquellen (4, 5) so steuert, dass die Verschiebungen des Optiksystems (1) in Bezug auf den festen Bezugswert ausgeglichen werden.

**Claims**

1. Microsurgical operation optical designation device comprising a tool holder plate (2) attached to an optical instrument (1), supporting two light sources (4, 5) offset with reference to the optical axis (10) of the optical instrument (1), said light sources being oriented so as to direct light beams (6, 7) in the direction of a target point, characterised in that the target point is in the patient reference point, that the optical instrument (1) and the tool holder plate (2) are attached to a mobile articulated support (3) with reference to a fixed reference point, that the orientation of the two sources (4, 5) is determined with reference to the optical axis of the optical instrument (1), that it comprises a computer and that it comprises, for each of the light sources (4, 5), an electric motor performing the angular movement of the light sources (4, 5) controlled by the computer in order to converge the beams (6, 7) at the target point (15), with the computer recalculating the orientation of each of the beams (6, 7) according to the movements of the support in the fixed reference point.

2. Microsurgical operation optical designation device according to claim 1 characterised in that the computer controls the motors performing the angular movement of the light sources (4, 5) so as to ensure the convergence of the light sources (6, 7) at the target point (15), the co-ordinates of which are known in the reference point of the field observed.

3. Microsurgical operation optical designation device according to claim 1 characterised in that it also comprises angular position sensors emitting a signal according to the orientation of each of the light sources and a computer to determine the depth of the target point (15).

4. Microsurgical operation optical designation device according to any of the above claims characterised in that it also comprises a computer controlling the light source (4, 5) positioning motors so as to compensate for the movements of the optical system (1) with reference to the fixed reference point.

Fig.1